(19) Europäisches Patentamt
European Patent Office
Office européen des brevets

(11) Veröffentlichungsnummer: **0 294 557 B1**

(12) **EUROPÄISCHE PATENTSCHRIFT**

(45) Veröffentlichungstag der Patentschrift: **08.07.92**

(51) Int. Cl.⁵: **C07C 33/20**, C07C 29/09, C07C 67/11, C07C 17/08, //C07C69/24,C07C22/04, C07C69/145

(21) Anmeldenummer: **88105670.9**

(22) Anmeldetag: **09.04.88**

# (54) Verfahren zur Herstellung von 3-Phenyl-2-methylpropanol und seinen p-substituierten Alkylderivaten.

(30) Priorität: **12.06.87 DE 3719693**

(43) Veröffentlichungstag der Anmeldung:
**14.12.88 Patentblatt 88/50**

(45) Bekanntmachung des Hinweises auf die Patenterteilung:
**08.07.92 Patentblatt 92/28**

(84) Benannte Vertragsstaaten:
**AT BE CH DE FR GB IT LI NL SE**

(56) Entgegenhaltungen:
**EP-A- 0 032 576**
**EP-A- 0 032 659**
**EP-A- 0 217 159**
**US-A- 4 546 208**

**CHEMICAL ABSTRACTS, Band 54, Nr. 3, 10. Februar 1960, Columbus, Ohio, USA TSUTOMO KUWATA et al. "3-(p-Isopropylphenyl)-2--methylpropionaldehyde" Zusammenfassung-Nr. 2 261i**

(73) Patentinhaber: **HÜLS AKTIENGESELLSCHAFT**
**Patentabteilung / PB 15 - Postfach 13 20**
**W-4370 Marl 1(DE)**

(72) Erfinder: **Kaufhold, Manfred, Dr.**
**Jasminweg 20**
**W-4370 Marl(DE)**

EP 0 294 557 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von 3-Phenyl-2-methylpropanol und seinen p-substituierten Alkylderivaten der Formel (1) durch peroxidkatalysierte Bromwasserstoffanlagerung an Methallylbenzol (2) bzw. an ein p-Alkyl-substituiertes Methallylbenzol (2), gegebenenfalls in Gegenwart des entsprechenden isomeren Isobutenylbenzols (3) bzw. des p-Alkyl-substituierten Isobutenylbenzols (3) und anschließende Umsetzung der Bromverbindungen (4) bzw. der Verbindungen (4) und (5) mit einem Alkalisalz einer Carbonsäure (6) zum Ester (7) und dessen Verseifung oder Umesterung mit einem niedrig siedenden Alkohol (8) zum gewünschten Alkohol (1). Gegebenenfalls setzt man nicht die reinen p-substituierten Alkylderivate von (2) bzw. (3) ein, sondern ein Gemisch, das neben den p-substitutierten Alkylderivaten die entsprechende m- und/oder o-substituierten Alkylderivate (2') und (3') enthält und trennt diese destillativ auf der Stufe der Ester (7) und (7') oder der Alkohole (1) und (1') als Vorlauf vor dem entsprechenden p-substituierten Isomeren (1) ab.

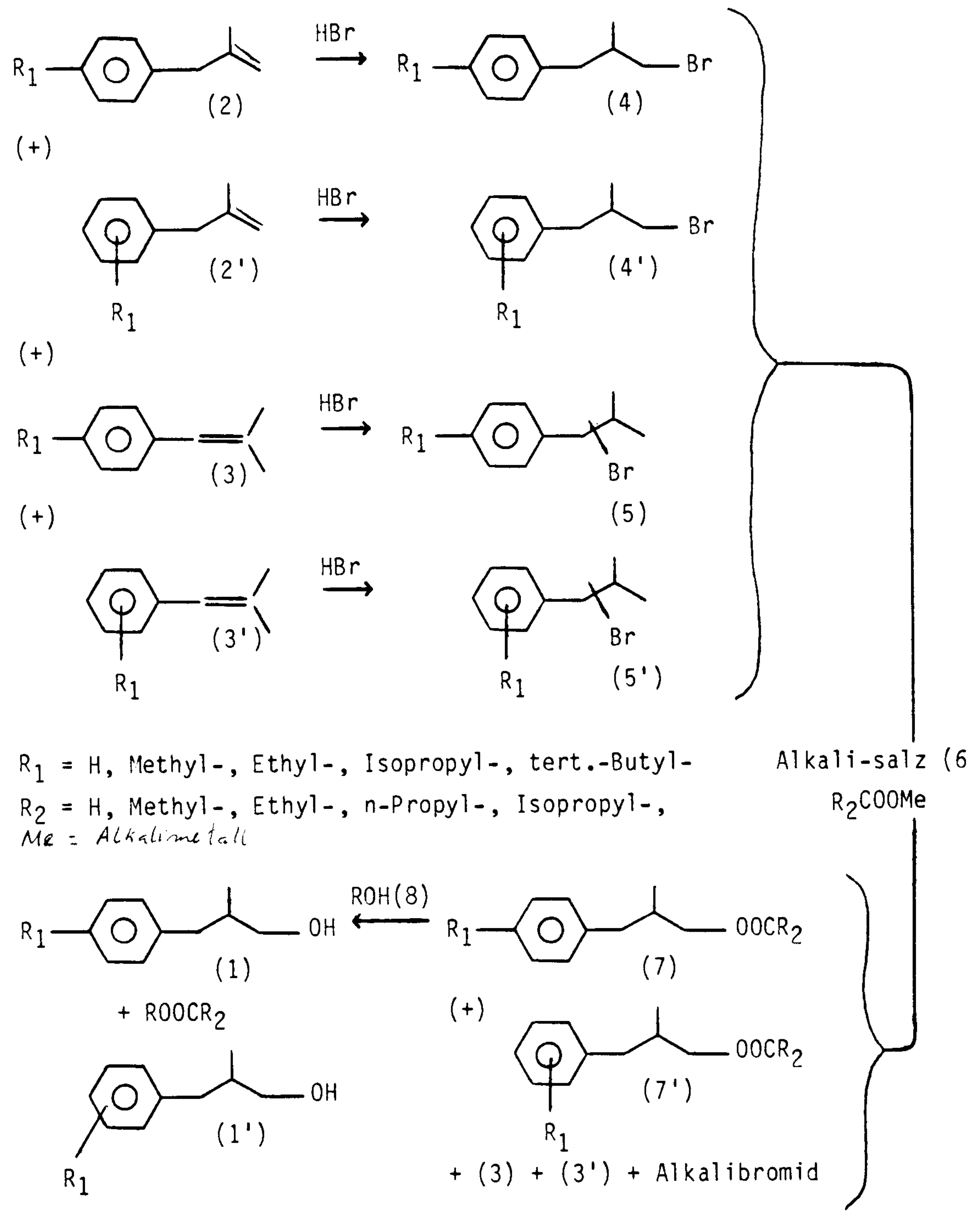


Synthesen von 3-Phenyl-2-methylpropanolen und ihren p-substituierten Alkylderivaten der Formel (1) sind bekannt.

Die JP-Patentanmeldung 6 973 (58) (Chem. Abstract 54 2261i (1960) beschreibt ein Verfahren zur Herstellung von 3-Phenyl-2-methylpropanol durch Hydrierung des entsprechenden Zimtaldehyds. Nachteilig bei diesem und ähnlichen Verfahren ist der Einsatz der kostspieligen 3-(p-Alkyl-phenyl)-2-methylpropenale, die aus den technisch schwer zugänglichen p-t-Alkylbenzaldehyden durch Aldolisierung mit Propionaldehyd hergestellt werden.

In der EP-PS 0 032 576 wird ein mehrstufiges Verfahren vorgeschlagen, bei dem Phenylpropanole in Gegenwart von Lewis-Säuren mit einem Alkylhalogenid alkyliert werden. Nachteil bei diesem Verfahren ist der sehr hohe Verbrauch an Lewis-Säuren. Zudem müssen die Phenylpropanole, die nicht leicht zugänglich sind, erst durch Aldolisierung von Benzaldehyd mit einem Alkanal, z. B. Propionaldehyd und anschließende

selektive Hydrierung hergestellt werden.

Die EP-PS 0 005 541 beschreibt ein Verfahren zur Herstellung von Morpholin- und Piperidin-Derivaten mit Hilfe von Bromiden, die der Struktur (4) entsprechen. Diese Bromide wurden aber aus den entsprechenden Alkoholen mit Phosphortribromid synthetisiert, die ihrerseits wieder in einer vielstufigen Synthese, ausgehend vom p-t-Butylbenzaldehyd, hergestellt wurden.

Alle bekannten Verfahren zur Herstellung von 3-Phenyl-2-methylpropanol und seinen p-substituierten Alkylderivaten gehen also von Benzaldehyd bzw. den teuren p-substituierten Benzaldehyden aus und benötigen teure Einsatzstoffe und/oder große Katalysatormengen, deren Vernichtung Umweltschutzprobleme mit sich bringt. Wünschenswert wäre ein Verfahren, mit dem man die Bromverbindung (4) bzw. ein Gemisch von (4) und (5) bzw. ein Gemisch von (4), (4′), (5) und (5′), ausgehend von preisgünstigen Chemikalien, in üblichen technischen Apparaturen herstellen, mit einem Alkalisalz einer Carbonsäure zu (7) bzw. zu einem Gemisch von (7) und (7′) umsetzen, diese Ester destillativ reinigen und anschließend aus den Estern durch Verseifung bzw. Umesterung den reinen Alkohol (1) herstellen könnte.

Es besteht somit ein großes Interesse an einem derartigen Verfahren, nach dem man bei geringem technischem Aufwand und ohne den Einsatz von teuren Reagenzien 3-Phenyl-2-methylpropanol und seine p-substituierten Alkylderivate herstellen kann, aus denen man durch Dehydrierung die entsprechenden Aldehyde gewinnt, die große wirtschaftliche Bedeutung als Riechstoffe, wie z. B. Lilial und Cyclamaldehyd haben und als Ausgangsverbindung für Fungizide dienen.

Die sich hieraus ergebende Aufgabe wird erfindungsgemäß entsprechend den Angaben der Patentansprüche gelöst.

Überraschenderweise erhält man 3-Phenyl-2-methylpropanol und seine p-substituierten Alkylderivate der allgemeinen Formel (1) in guten Ausbeuten und mit hoher Reinheit hinsichtlich der Isomeren von 95 bis 98 % nach gaschromatographischer Analyse, indem man an Methallylbenzol bzw. an ein p-Alkyl-substituiertes Methallylbenzol (2) Bromwasserstoff in Gegenwart vn Peroxiden addiert, die entstandene Bromverbindung (4) mit einem Alkalisalz einer Carbonsäure zu dem entsprechenden Ester umsetzt und diesen Ester verseift oder zum Alkohol (1) umestert. Dieser Effekt war nicht zu erwarten, da die Bromwasserstoffaddition eine Reihe von Nebenverbindungen liefert, die ebenfalls mit dem Alkalisalz einer Carbonsäure reagieren und zu Verunreinigungen im gewünschten Produkt führen sollten. An die Reinheit des eingesetzten Methallylbenzols (2) werden keine hohen Anforderungen gestellt.

Ferner wurde überraschenderweise gefunden, daß der Gehalt an dem entsprechenden isomeren beta-Olefin, dem Isobutenylbenzol bzw. dem p-Alkyl-substituierten Isobutenylbenzol (3) im alpha-Olefin, dem gegebenenfalls p-Alkyl-substituierten Methallylbenzol, beliebig hoch sein kann, ohne daß dadurch die Reinheit im Endprodukt verringert wird, d. h. man kann ein Olefingemisch von (2) und (3) einsetzen und erhält im Endeffekt nur das Umsetzungsprodukt von (2).

Darüber hinaus wurde gefunden, daß man überraschenderweise nicht reine p-Alkyl-substituierte Methallyl- und entsprechende Isobutenylbenzole einsetzen muß, sondern die Isomerengemische einsetzen kann, wie sie bei der technischen Herstellung einer Aromatenalkylierung anfallen. Je nach Reaktionsbedingungen beträgt der Gehalt an p-substituierten Alkylaromaten nur 70 bis 80 % und der Rest sind die entsprechenden o-und/oder m-substituierten Verbindungen. Es ist ein besonderer Vorteil dieses erfindungsgemäßen Verfahrens, daß man also auch o-, m- und p-Isomerengemische einsetzen und auf der Stufe der Ester oder der Alkohole die gewünschte p-substituierte Verbindung in reiner Form abtrennen kann.

Die Herstellung der Ausgangsprodukte erfolgt beispielsweise durch Umsetzung eines Aromaten, z. B. Benzol, Toluol, Ethylbenzol, Cumol, tert.-Butylbenzol u. a. mit Methallylchlorid in Gegenwart von Schwefelsäure als Katalysator zu Neophylchlorid ( = 2-Methyl-2-Phenylpropylchlorid) bzw. p-Alkyl-substituiertem Neophylchlorid. Diese können durch Destillation oder Umkristallisieren gereinigt oder auch als Rohprodukt einer thermischen Spaltung unterworfen werden, wie sie z. B. in der US-PS 2 454 779 beschrieben ist.

Bei dem Verfahren dieser Patentschrift erfolgt die Spaltung der Chlorverbindungen in Gegenwart von Alkalisalzen von Carbonsäuren und dadurch bedingt liegt das Isomerenverhältnis von alpha/beta-Olefin bei 2 : 1 bis 1 : 1.

Zur praktischen Durchführung ist folgendes wichtig:

Die Anlagerung von Bromwasserstoff an das Einsatzolefin erfolgt bei 0 bis 20 °C in Gegenwart von einem Peroxid, z. B. Dibenzoylperoxid und einem unpolaren Lösemittel, wie z. B. Hexan, wie für andere Olefine in der Literatur beschrieben: "Methoden der Organischen Chemie", Houben-Weyl, Georg-Thieme-Verlag Stuttgart (1960), Band V/4, Seite 111.

Nach dem Abdestillieren des Lösemittels wird das rohe Umsetzungsprodukt aufdestilliert oder direkt weitverarbeitet.

Dazu wird das Alkalisalz einer Carbonsäure, vorzugsweise mit 1 bis 4 C-Atomen, vorzugsweise Natrium- oder Kaliumacetat vorgelegt, gegebenenfalls noch Säure und/oder ein polares Lösemittel, wie z. B.

Dimethylformamid, Acetonitril, Dioxan, Tetrahydrufuran, Dimethoxyethan u. a. zugegeben, auf 100 °C bis 200 °C bzw. bis Rückflußtemperatur erwärmt und das rohe Bromidgemisch zudosiert. Dann wird 1 bis 10 Stunden, vorzugsweise 2 bis 4 Stunden, unter Rühren auf 100 bis 200 °C, vorzugsweise auf 120 bis 180 °C bzw. auf Rückflußtemperatur erhitzt. Das Molverhältnis Bromverbindung zu Alkalisalz beträgt 1 : 1 bis 1 : 10, vorzugsweise 1 : 1,01 bis 1 : 2.

Nach der Umsetzung wird das gegebenenfalls zugesetzte polare Lösemittel abdestilliert, abgekühlt und das gebildete Alkalibromid mit Wasser ausgewaschen. Die Ölphase wird dann destillativ aufgearbeitet.

Den erhaltenen Ester verseift man oder estert mit einem niedrigsiedenden Alkohol um zum gewünschten Alkohol (1). Als niedrigsiedende Alkohole sind $C_1$- bis $C_6$-Alkohole geeignet.

Die folgenden Beispiele sollen das erfindungsgemäße Verfahren näher erläutern.

## Beispiel 1

Man benutzt eine Glasapparatur, die aus einem Dreihalskolben mit Gaseinleitungsrohr, Rührer, Thermometer, Tropftrichter und Rückflußkühler besteht.

Man setzt ein:

198 g (= 1,5 Mol) Methallylbenzol (99,8 %ig)
666 g n-Hexan
10,8 g Dibenzoylperoxid (70 %ig)

Die Lösung dieser Substanzen wird auf 10 °C gekühlt und bis zur Sättigung gasförmiger Bromwasserstoff eingeleitet.

Das Lösemittel wird abdestilliert, bis eine Sumpftemperatur von 104 °C bei 300 mbar erreicht ist und der rohe Destillationsrückstand, 318 g, wird weiterverarbeitet. Nach gaschromatographischer Analyse liegt der Gehalt an 3-Phenyl-2-methyl-1-brompropan bei ca. 82 %.

Zur Umsetzung des rohen Bromids aus Stufe 1 benutzt man die oben beschriebene Apparatur, entfernt das Gaseinleitungsrohr und legt vor:

148,0 g (= 1,68 Mol) Natriumisobutyrat
17,6 g (= 0,2 Mol) Isobuttersäure

Das Gemisch wird auf 113 °C erhitzt, das rohe Bromid aus Stufe 1, 318 g, zugegeben und 8,5 h unter Rühren auf 114 bis 117 °C erhitzt.

Nach dem Abkühlen wird das gebildete Natriumbromid mit Wasser ausgewaschen und die Ölphase destillativ aufgearbeitet.

Es entstehen 212 g Ester mit einer Reinheit von 97 % (Siedebereich bei 13 mbar 118 bis 120 °C), d. h. die Ausbeute auf eingesetztes Methallylbenzol beträgt ca. 65 %.

120 g (= 1,5 Mol) Natronlauge (50 %ig) werden unter Rühren auf 120 °C erwärmt, 198,2 g (= 1,0 Mol) des Esters (97 %ig) zugetropft und anschließend 6 h lang am Rückfluß zum Sieden erhitzt. Nach dem Abkühlen wird mit 400 ml Wasser verdünnt und mit 500 ml Diethylether extrahiert. Die Etherlösung wird mit Wasser neutral gewaschen und anschließend destillativ aufgearbeitet. Die Destillation liefert in einem Siedebereich von 107 bis 112 °C bei 13 mbar das 3-Phenyl-2-methylpropanol-1 mit einer Reinheit von 98 %. Die Alkoholausbeute, bezogen auf eingesetzten Ester, beträgt 94 % der Theorie.

## Beispiel 2

Man benutzt die im Beispiel 1 beschriebene Glasapparatur und setzt anstelle von Methallylbenzol ein Gemisch ein, das zu 66,3 % aus Methallylbenzol und zu 31,1 % aus Isobutenylbenzol besteht. Die peroxidkatalysierte Bromwasserstoffaddition und die anschließende Umsetzung mit Natriumisobutyrat werden in gleicher Weise durchgeführt wie im Beispiel 1 beschrieben.

Die Ausbeute an gebildetem Ester liegt bei 63 %; die Reinheit des Esters ist nach gaschromatographischer Analyse ca. 97 %. Ca. 88 % des eingesetzten Isobutenylbenzol werden zurückgewonnen, d. h. werden aus dem Bromidgemisch zurückgebildet.

Die alkalische Verseifung des Esters wird, wie in Beispiel 1 beschrieben, durchgeführt und liefert den Alkohol (Reinheit 98 %) in einer Ausbeute von 93 % der Theorie.

## Beispiel 3

Man benutzt die im Beispiel 1 beschriebene Glasapparatur und setzt anstelle von Methallylbenzol folgendes Gemisch ein:

384,7 g (= 2 Mol) Olefingemisch 97,9 %ig

mit p-t-Butylmethallylbenzol
70,1 % = 1,40 Mol und
mit p-t-Butylisobutenylbenzol
27,8 % = 0,56 Mol

Die peroxidkatalysierte Bromwasserstoffaddition erfolgt wie im Beispiel 1 beschrieben. Nach NMR-Spektrum liegt das Verhältnis vom primären Bromid zu den übrigen Bromiden bei 7 : 3 im Destillationsrückstand, 643 g, nach Abstoppen der Hauptmenge des Lösemittels, 538 g.

Zur Esterherstellung werden eingesetzt:
643 g des rohen Bromidgemisches
193 g (= 2,3 Mol) Natriumacetat
312 g Dimethylformamid

Das Gemisch wird unter Rühren und bei Abnahme von Leichtsiedern wie Hexan, 102 g, 4,5 h lang auf 90 bis 160 °C erhitzt.

Die Aufarbeitung erfolgt wie im Beispiel 1 beschrieben und liefert in einem Siedebereich von 150 bis 155 °C bei 13 mbar den Essigsäureester des 3-(4-tert.-Butylphenyl)-2-methyl-propanol-1 mit einer Reinheit von 98 %.

Ausbeute an Ester 209 g (= 0,84 Mol)

Zurückgewonnene Olefine: p-t-Butylmethallylbenzol 59,3 g (= 0,315 Mol). Verbraucht wurden demnach 203 g (= 1,08 Mol).

p-t-Butylisobutenylbenzol: zurückgewonnen 52 g (= 0,28 Mol.
Verbraucht wurden demnach 52 g (= 0,28 Mol).

Die Ausbeute an Ester, auf verbrauchtes alpha-Olefin bezogen, errechnet sich zu 78 %.

Die alkalische Verseifung des Essigsäureesters des 3-(4-t-Butylphenyl)-2-methylpropanols-1 erfolgt in analoger Weise wie beim Beispiel 1 beschrieben und liefert den entsprechenden Alkohol in einem Siedebereich von 142 bis 146 °C bei 13 mbar mit einer Reinheit von 98,2 % und in einer Ausbeute von 93 %, bezogen auf eingesetzten Ester.

Beispiel 4

Man benutzt die im Beispiel 1 beschriebene Glasapparatur und setzt anstelle von Methallylbenzol folgendes Isomerengemisch ein:
377 g (= 1,93 Mol) Olefingemisch 96,6 %ig
mit folgenden Isomerengehalten

| | |
|---|---|
| p-t-Butylmethallylbenzol | 57,0 % = 1,14 Mol |
| p-t-Butylisobutenylbenzol | 22,7 % = 0,45 Mol |
| m-t-Butylmethallylbenzol | 12,1 % = 0,24 Mol |
| m-t-Butylisobutenylbenzol | 4,8 % = 0,10 Mol |

Die peroxidkatalysierte Bromwasserstoffaddition erfolgt wie im Beispiel 1 beschrieben. Nach Abdestillieren der Hauptmenge an Lösemittel, 540 g, werden 652 g Rohprodukt erhalten, in dem nach NMR-Spektrum die Bromverbindungen im Verhältnis primäres Bromid zu den übrigen Bromiden wie 2 : 1 vorliegt.

Die Esterherstellung erfolgt wie im Beispiel 3 beschrieben.

Die Aufarbeitung wird wie im Beispiel 1 beschrieben durchgeführt und liefert folgende Produkte:

| | |
|---|---|
| 3(4-t-Butylphenyl)-2-methyl-propylacetat (Reinheit: 96 %) | 199 g = 0,80 Mol. |
| 3(3-t-Butylphenyl)-2-methylpropylacetat | 32 g = 0,13 Mol |
| p-t-Butylmethallylbenzol | 55 g = 0,29 Mol |
| p-t-Butylisobutenylbenzol | 41 g = 0,22 Mol |
| m-t-Butylmethallylbenzol | 17 g = 0,09 Mol |
| m-t-Butylisobutenylbenzol | 5 g = 0,03 Mol |

Die Ausbeute an gewünschtem Ester, bezogen auf verbrauchtes alpha-Olefin, errechnet sich zu 94 %.

83 g (= 0,316 Mol) p-t-Butylphenyl-2-methyl-propylacetat (ca. 95 %ig) werden mit 20 g Methanol und 1,1 g einer 30 %igen Lösung von Natriummethylat in Methanol versetzt und zum Sieden erhitzt. Der dabei entstehende Essigsäuremethylester wird über eine 50 cm lange Kolonne abdestilliert und unter Anlegen von

Vakuum, 13 mbar, wird der gebildete Alkohol, das 3(4-t-Butylphenyl)-2-methypropanol, direkt überdestilliert. Siedepunkt: ca. 145 °C bei 13 mbar; Reinheit: 98,5 %. Ausbeute 61,2 g (= 0,296 Mol), das sind 94 %, bezogen auf Einsatz.

Beispiel 5

Man benutzt die im Beispiel 1 beschriebene Glasapparatur und setzt anstelle von Methallylbenzol folgendes Isomerengemisch ein:
522,9 g (= 2,8 Mol) Olefingemisch (93,3 %ig) mit folgenden Isomerengehalten:

| | |
|---|---|
| p-Isopropylmethallylbenzol | 57,2 % = 1,71 Mol |
| p-Isopropylisobutenylbenzol | 22,3 % = 0,70 Mol |
| m-Isopropylmethallylbenzol | 10,0 % = 0,29 Mol |
| m-Isopropylisobutenylbenzol | 3,8 % = 0,10 Mol |

Die peroxidkatalysierte Bromwasserstoffaddition erfolgt wie im Beispiel 1 beschrieben und die Esterherstellung wie im Beispiel 3 beschrieben.

Die Aufarbeitung liefert analoge Ergebnisse wie im Beispiel 4: Der bei 140 bis 148 °C bei 14 mbar siedende Ester hat eine Reinheit von 96 %. 77 % des eingesetzten alpha-Olefins werden verbraucht. Bezogen auf verbrauchtes alpha-Olefin beträgt die Ausbeute an Ester 92 %.

Die Umesterung des Essigsäureesters erfolgt in analoger Weise wie in Beispiel 4 beschrieben und ergibt den entsprechenden Alkohol in einem Siedebereich von 131 bis 135 °C bei 13 mbar (Reinheit 97,5 %) mit einer Ausbeute von 96 %, bezogen auf eingesetzten Ester.

**Patentansprüche**

**1.** Verfahren zur Herstellung von 3-Phenyl-2-methyl-propanol und seinen p-substituierten Alkylderivaten, dadurch gekennzeichnet,
daß man Bromwasserstoff peroxidkatalysiert an gegebenenfalls p-Alkyl-substituierte Methallylbenzole (2) bzw. an einem Gemisch aus gegebenenfalls p-Alkylsubstituiertem Methallylbenzol (alpha- Olefin) (2) und dem entsprechenden beta-Isomeren, dem gegebenenfalls p-Alkylsubstituiertem Isobutenylbenzol (3) anlagert, die erhaltene Bromverbindung mit einem Alkalisalz einer Carbonsäure, dem gegebenenfalls eine Säure und/oder ein polares Lösemittel zugesetzt werden, bei Temperatur von 100 °C bis 200 °C bzw. bis Rückflußtemperatur und Molverhältnissen Bromverbindung zu Alkalisalz bis 1 : 10, umsetzt und den erhaltenen Ester (7) zum entsprechenden Alkohol (1) verseift bzw. mit einem niedrig siedenden Alkohol (8) zu (1) umestert,

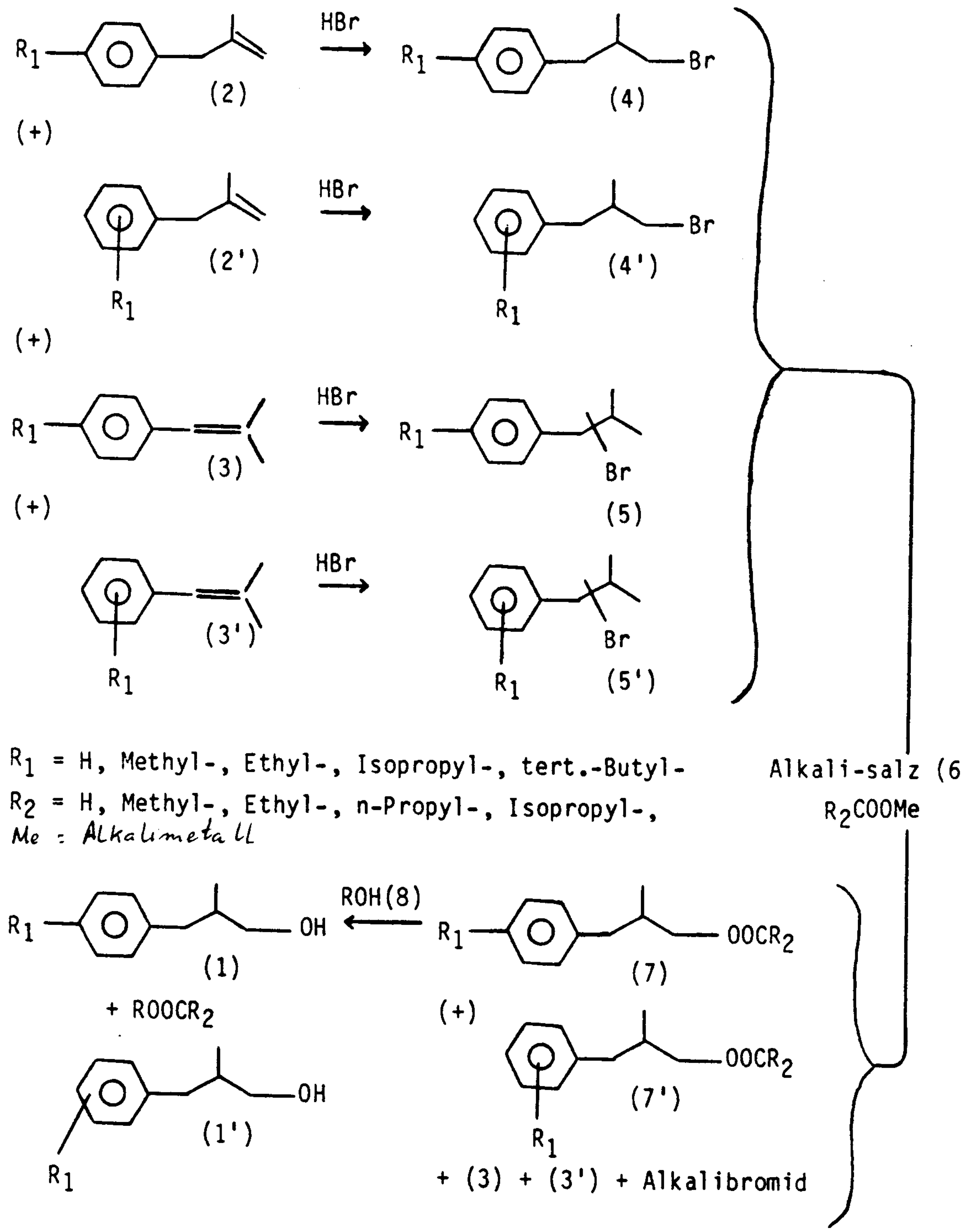


$R_1$ = H, Methyl-, Ethyl-, Isopropyl-, tert.-Butyl-
$R_2$ = H, Methyl-, Ethyl-, n-Propyl-, Isopropyl-,
Me : Alkalimetall

2. Verfahren nach Anspruch 1,
dadurch gekennzeichnet,
daß man bei der Bromwasserstoffaddition zusätzlich zu dem Gemisch der alpha- und beta-Olefine (2) und (3) auch die entsprechenden in m- und/oder in o-Stellung substituierten alpha- und beta-Olefine einsetzt.

3. Verfahren nach den Ansprüchen 1 und 2,
dadurch gekennzeichnet,
daß man Bromwasserstoff bei 0 bis 20 °C anlagert.

4. Verfahren nach einem der Ansprüche 1 bis 3, dadurch gekennzeichnet, daß man die Bromverbindung mit einem Alkalisalz einer $C_1$-$C_4$-Carbonsäure, vorzugsweise mit Natrium- oder Kaliumacetat, umsetzt.

5. Verfahren nach einem der Ansprüche 1 bis 4, dadurch gekennzeichnet, daß man die Umsetzung der Bromverbindung mit dem Alkalisalz einer Carbonsäure bei Temperaturen von 100 bis 200 °C, vorzugsweise 120 bis 180 °C durchführt.

6. Verfahren nach einem der Ansprüche 1 bis 5, dadurch gekennzeichnet, daß man ein Molverhältnis Bromverbindung zu Alkalisalz von 1 : 1,01 bis 1 : 2 einsetzt.

## Claims

1. A process for the preparation of 3-phenyl-2-methyl propanol and its p-substituted alkyl derivatives, characterised in that hydrogen bromide is added peroxide catalysed to possibly p-alkyl-substituted methallyl benzenes (2) or to a mixture of possibly p-alkyl-substituted methallyl benzene (alpha-olefin) (2) and the corresponding beta-isomer, the possibly p-alkyl-substituted isobutyl benzene (3), the bromide compound obtained is reacted with an alkali salt of a carboxylic acid, to which if necesary an acid and/or a polar solvent is added, at a temperature of 100 °C or 200 °C or to reflux temperature and with bromine compound/alkali salt molar ratios of up to 1 : 10, and the ester (7) obtained is saponified to give the corresponding alcohol (1) or re-esterified with a low-boiling alcohol (8) to give (1),

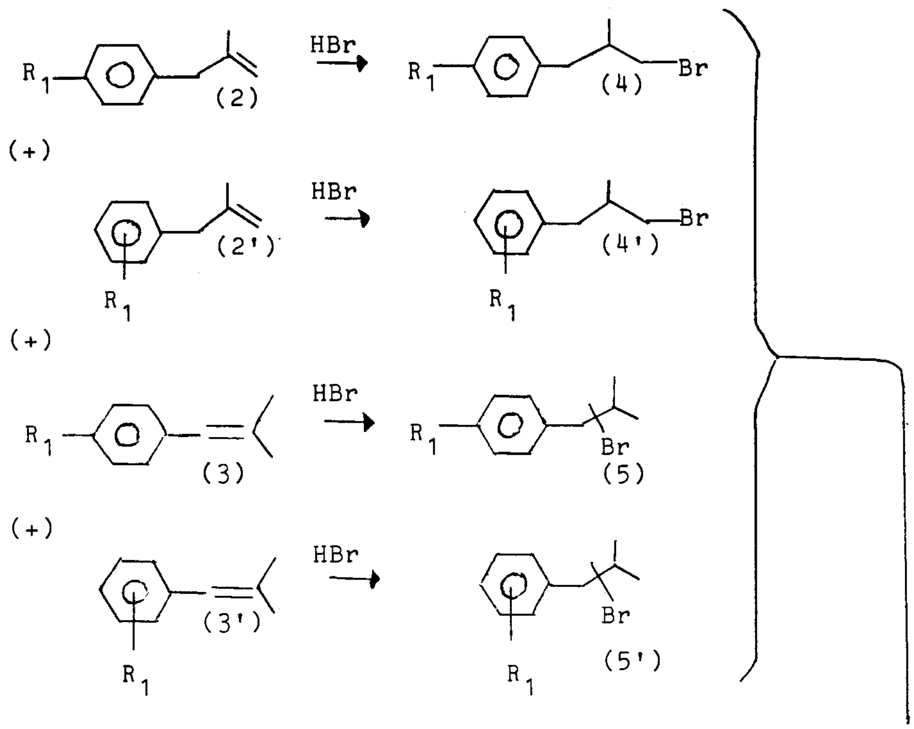


$R_1$ = H, methyl-, ethyl-, isopropyl-, tert.-butyl-

$R_2$ = H, methyl-, ethyl-, n-propyl-, isopropyl-,

Me = alkali metal

alkali salt (6)

$R_2COOMe$

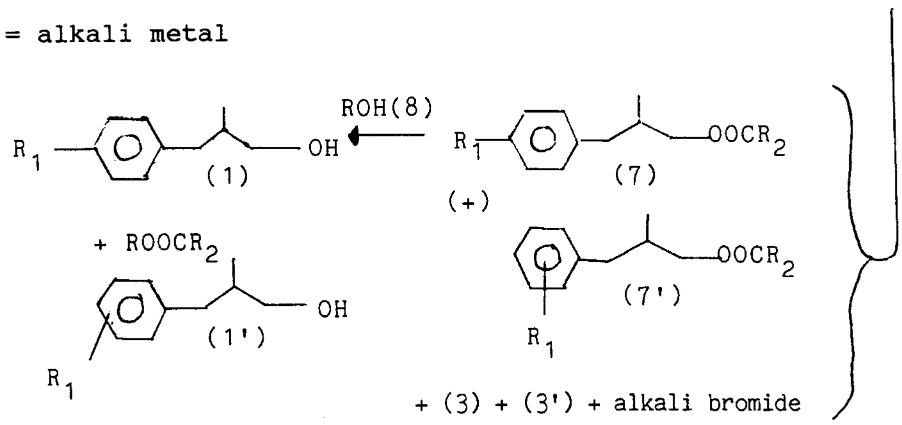


2. A process according to claim 1, characterized in that as well as the mixture of the alpha- and beta-olefins (2) and (3), the corresponding alpha- and beta-olefins substituted in the mand/or o-position are added to the hydrogen bromide.

3. A process according to claims 1 and 2, characterized in that the addition is made to the hydrogen bromide at 0 to 20 ° C.

4. A process according to one of claims 1 to 3, characterized in that the bromine compound is reacted with an alkali salt of a $C_1$-$C_4$ carboxylic acid.

5. A process according to one of claims 1 to 4, characterized in that the bromine compound is reacted with the alkali salt of a carboxylic acid at temperatures of 100 to 200 ° C, preferably 120 to 180 ° C.

6. A process according to one of claims 1 to 5, characterized in that a bromine compound/alkali salt molar ratio of 1 : 1.01 to 1 : 2 is used.

## Revendications

1. Procédé pour la préparation du 3-phényl-2-méthyl-propanol et de ses dérivés alkylés substitués en position para, caractérisé en ce que l'on fixe par addition, catalysée par un peroxyde, de l'acide bromhydrique sur des méthallylbenzènes (2) éventuellement substitués par des groupes alkyle en position para ou sur un mélange de méthallylbenzène éventuellement substitué par un groupe alkyle en position para (3) (alpha-oléfine) et de i'isomère bêta correspondant, l'isobuténylbenzène (3) éventuellement substitué par un groupe alkyle en position para, on fait réagir le composé bromé obtenu avec un sel alcalin d'un acide carboxylique auquel est éventuellement ajouté un acide et/ou un solvant polaire, à une température de 100 à 200 °C ou à la température de reflux et à des rapports molaires du composé bromé au sel alcalin allant jusqu'à 1:10 et on saponifie l'ester (7) obtenu en l'alcool correspondant ou on le transestérifie avec un alcool (8) à bas point d'ébullition, pour aboutir au composé (1),

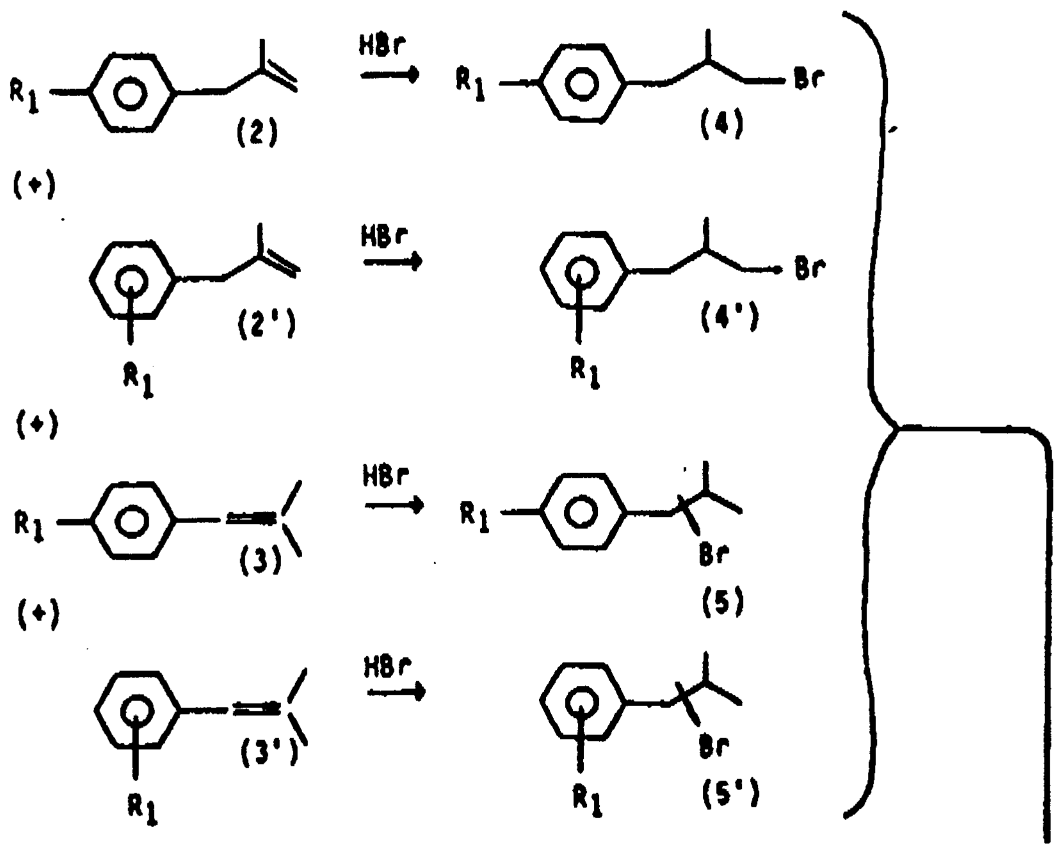


$R_1$ = H, méthyle, éthyle, isopropyle, tert.-butyle, sel alcalin (6 $R_2COOMe$)
$R_2$ = H, méthyle, éthyle, n-Propyle, isopropyle,
Me = métal alcalin

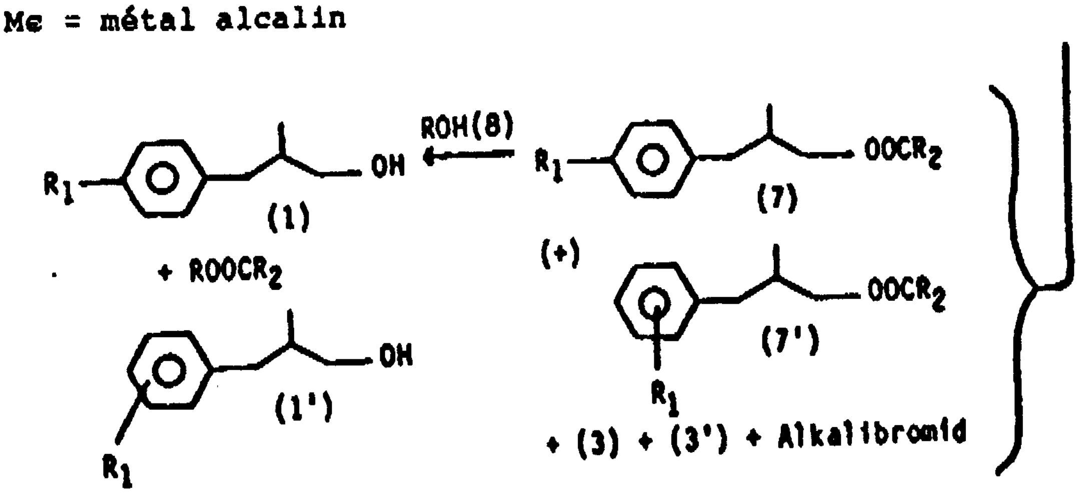


**2.** Procédé selon la revendication 1, caractérisé en ce que l'on utilise en outre dans l'addition d'acide bromhydrique au mélange des alphaet bêta-oléfines (2) et (3) également les alpha- et bêta-oléfines correspondantes, substltuées en position(s) méta et/ou ortho.

**3.** Procédé selon la revendication 1 ou 2, caractérisé en ce que l'on fixe par addition l'acide bromhydrique à 0-20 °C.

**4.** Procédé selon l'une quelconque des revendications 1 à 3, caractérisé en ce que l'on fait réagir le composé bromé avec un sel alcalin d'un acide carboxylique en $C_1$-$C_4$, de préférence avec de l'acétate de sodium ou de potassium.

**5.** Procédé selon l'une quelconque des revendications 1 à 4, caractérisé en ce que l'on effectue la réaction du composé bromé avec le sel alcalin d'un acide carboxylique à des températures de 100 à 200 °C, de préférence de 120 à 180 °C.

**6.** Procédé selon l'une quelconque des revendications 1 à 5, caractérisé en ce que l'on utilise un rapport molaire du composé bromé au sel alcalin allant de 1:0,1 à 1:2.